(19) Europäisches Patentamt
European Patent Office
Office européen des brevets

(11) Veröffentlichungsnummer: **0 244 509**
**B1**

(12) # EUROPÄISCHE PATENTSCHRIFT

(45) Veröffentlichungstag der Patentschrift: **31.10.90**

(51) Int. Cl.⁵: **A01N 43/40,** C07D 213/65

(21) Anmeldenummer: **86116270.9**

(22) Anmeldetag: **24.11.86**

(54) **Mittel zur Bekämpfung von Mikroben.**

(30) Priorität: **30.04.86 DE 3614706**

(43) Veröffentlichungstag der Anmeldung:
**11.11.87 Patentblatt 87/46**

(45) Bekanntmachung des Hinweises auf die Patenterteilung:
**31.10.90 Patentblatt 90/44**

(84) Benannte Vertragsstaaten:
**AT BE CH DE ES FR GB GR IT LI NL SE**

(56) Entgegenhaltungen:
**EP-A- 0 207 411**
**DE-A- 2 215 503**
**DE-A- 2 403 429**
**DE-C- 532 396**
**SU-A- 178 379**
**US-A- 2 728 775**
**US-A- 4 220 577**

(73) Patentinhaber: **BASF Aktiengesellschaft,**
**Carl-Bosch-Strasse 38, D-6700 Ludwigshafen(DE)**

(72) Erfinder: **Pommer, Ernst-Heinrich, Dr., Berliner Platz 7,**
**D-6703 Limburgerhof(DE)**
Erfinder: **Ammermann, Eberhard, Dr., Sachsenstrasse 3,**
**D-6700 Ludwigshafen(DE)**

**EP 0 244 509 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft Mittel zur Bekämpfung von Mikroben, die O-substituierte 3-Oxypyridiniumsalze enthalten, sowie Verfahren zur Bekämpfung von Mikroben.

Es wurde gefunden, daß Mittel zur Bekämpfung von Algen, Bakterien oder Pilzen außer bei Pflanzen, die ein O-substituiertes 3-Oxypyridiniumsalz der Formel

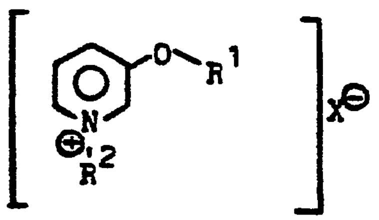 I

enthalten, in der

$R^1$ einen gegebenenfalls durch Alkoxy, Cycloalkoxy, Phenylalkoxy oder Halogen substituierten Alkyl-, Alkenyl- oder Alkinylrest mit insgesamt 8 - 20 C-Atomen

$R^2$ einen gegebenenfalls durch Halogen substituierten Alkyl-, Alkenyl- oder Alkinylrest mit 1 bzw. 2 oder 3 bis 8 Kohlenstoffatomen oder eine der Gruppen

$$-(CH_2)_{1-2}-Ar, \quad -(CH_2)_{2-5}OCH_2\underset{R^3}{\underset{|}{C}}HCH_2Ar$$

oder $-(CH_2)_{2-6}-O-Ar$ bedeutet, wobei Ar für 1- und 2-Naphthyl, Biphenyl oder Phenyl steht und der Phenylrest durch F, Cl, Br, $NO_2$, $CF_3$, CN, $C_{1-13}$-Alkyl, $C_{2-4}$-Alkenyl oder $C_{1-2}$-Alkoxy substituiert sein kann, und $R^3$ H, $C_1$-$C_2$-Alkyl, $C_3$-$C_5$-Alkenyl oder $C_1$-$C_5$-Alkoxy bedeutet und

X-$F^-$, $Cl^-$, $Br^-$ oder $I^-$ oder ein Anion, das Äquivalent einer Säure ist, bedeutet, eine gute mikrobizide Wirksamkeit zeigen.

$R^1$ bedeutet beispielsweise gerades oder verzweigtes, Octyl, Decyl, Undecyl, Dodecyl, Tridecyl, Tetradecyl, Pentadecyl, Hexadecyl, Heptadecyl, Octadecyl oder die Gruppe $-C_{2-10}$Alkyl-O-$C_{5-18}$Alkyl, -O-Cyclohexyl, -O-$C_3$-$C_5$-Alkylphenyl mit insgesamt 10 - 20 C-Atomen

$R^2$ bedeutet beispielsweise $C_1$-$C_4$-Alkyl, Methyl, Ethyl, Propyl, Isopropyl, n-Butyl, Isobutyl, Allyl, 2-Chlorpropenyl, 2-Brompropenyl, 3-Chlorpropenyl, 2-Buten-1-yl, 2-Methylpropenyl, 3-Chlor-2-buten-1-yl, Propargyl oder die Reste $-(CH_2)_{1-2}$-Ar oder $-(CH_2)_{2-6}$OAr,
wobei Ar, für Phenyl, einfach bis dreifach durch Halogen substituiertes Phenyl, 4-Fluorphenyl, 3- oder 4-Chlorphenyl, 4-Bromphenyl, 3,4-Dichlorphenyl, 2,4-Dichlorphenyl, 2,6-Dichlorphenyl, 2,4,6-Trichlorphenyl steht und

$X^\ominus$ bedeutet $Cl^\ominus$, $Br^\ominus$, $I^\ominus$ oder ein Anion, das ein Äquivalent einer Säure ist wie Phenylsulfonsäure, p-Methylphenylsulfonsäure oder ein Äquivalent des Schwefelsäure-Anions.

Man erhält die O-substituierten 3-Oxypyridiniumsalze der Formel I durch Umsetzung von Verbindungen der Formel

$R^2$XII,

in der $R^2$ und X die oben angegebenen Bedeutungen haben mit einem O-substituierten 3-Oxypyridin-Derivat der Formel

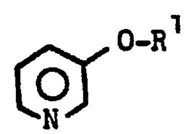 III,

in der $R^1$ die obengenannte Bedeutung hat.

Die Reaktion wird ohne Verdünnungsmittel in Substanz oder gegebenenfalls in Gegenwart eines inerten Lösungs- oder Verdünnungsmittels bei einer Temperatur zwischen 20 und 150 °C vorzugsweise zwi-

schen 50 und 150 °C durchgeführt. Der Ausgangsstoff der Formel III wird zweckmäßig mit einer bis zu 10fachen Menge (molar) des Alkylierungsmittels der Formel II bezogen auf den Ausgangsstoff III umgesetzt.

Als bevorzugte, gegenüber den Reaktionsteilnehmern inerte Lösungsmittel oder Verdünnungsmittel können beispielsweise aliphatische oder aromatische gegebenenfalls halogenierte Kohlenwasserstoffe, wie Pentan, Cyclohexan, Heptan, Benzol, Toluol, Xylol, Chlorbenzol, Dichlorbenzole, aliphatische Ketone, wie Aceton, Methylethylketon, Diethylketon oder Cyclopentanon, Ether wie Diethylether, Methyl-tert.-butylether, Dimethoxyethan, Tetrahydrofuran oder Dioxan, Ester wie Essigsäureethylester, Nitrile wie Acetonitril, Amide wie Dimethylformamid, Dimethylacetamid und N-Methylpyrrolidon, oder Gemische dieser Lösungsmittel verwendet werden.

Die Ausgangsstoffe der Formel II und III sind weitgehend bekannt und z. T. kommerziell zugänglich, bzw. sie lassen sich analog zu literaturbekannten Beispielen herstellen (für III z. B. entsprechend Chem. Ber. 116, 2394 (1983).

Die neuen O-substituierten 3-Oxypyridiniumsalze besitzen gegebenenfalls in $R^1$ und/oder $R^2$ ein chirales Kohlenstoffatom, wenn $R^3$ nicht gleich Wasserstoff ist. Nach den üblichen Methoden können die optisch reinen Enantiomeren bzw. die Diastereomeren erhalten werden. Die vorliegende Erfindung erfaßt sowohl diese Verbindungen in reiner Form als auch ihre Mischungen. Es sind sowohl die reinen Enantiomeren bzw. die einheitlichen Diastereomeren als auch die bei der Synthese üblicherweise anfallenden Mischungen wirksam.

Das folgende Beispiel erläutert die Herstellung der neuen Verbindungen:

## Herstellungsbeispiel

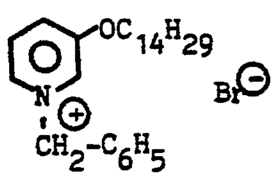

### a) Ausgangsmaterial

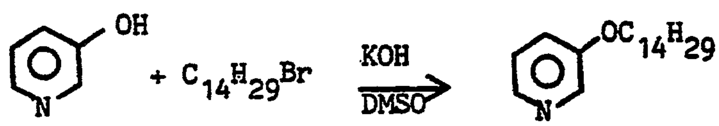


A

100 g (1,04 mol) 3-Hydroxypyridin werden in 500 ml Dimethylsulfoxid (DMSO) mit 87,4 g (1,56 mol) KOH-Pulver unter Stickstoff bei Raumtemperatur (20°C) gerührt. Nach ca. 30 Min. trägt man 360 g (1,3 mol) Tetradecylbromid tropfenweise ein und rührt 4 h bei Raumtemperatur nach. Das Reaktionsgemisch wird in 1 l Wasser gegossen und dreimal mit je 500 ml Methylenchlorid extrahiert. Die vereinigten Extrakte werden zweimal mit 1 l Wasser gewaschen, mit $Na_2SO_4$ getrocknet und eingedampft. Filtration über Kieselgel mit n-Pentan und Methylchlorid führt zu 144 g Produkt A , Fp. 36°C.

### b) Pyridiniumsalzbildung

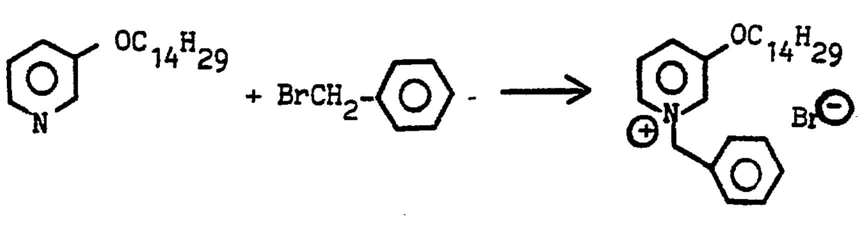

A

B

11,5 g (O,O4 mol) 3-Tetradecyloxypyridin A werden mit 5 ml (O,O4 mol) Benzylbromid 2O Min. bei 1OO - 12O°C gerührt. Nach Abkühlen auf Raumtemperatur wird in Pentan suspendiert, abgesaugt, mit Pentan nachgewaschen und getrocknet: 16,8 g Pyridiniumsalz B, Fp. 129°C (Verbindung Nr. 23).

Die in der folgenden Tabelle aufgelisteten Verbindungen sind auf entsprechende Weise herstellbar:

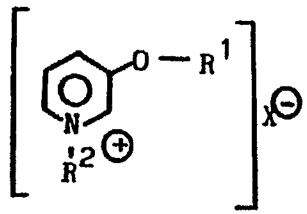

| Nr. | $R^1$ | $R^2$ | X | Fp. [°C] |
|---|---|---|---|---|
| 1 | $n-C_{10}H_{21}$ | $CH_3$ | Cl | |
| 2 | $n-C_{10}H_{21}$ | $CH_3$ | Br | |
| 3 | $n-C_{10}H_{21}$ | $CH_3$ | I | |
| 4 | $n-C_{12}H_{25}$ | $CH_3$ | Br | |
| 5 | $n-C_{12}H_{25}$ | $CH_3$ | I | 64 - 71 |
| 6 | $n-C_{12}H_{25}$ | $-CH_2-CH=CH_2$ | Br | 69 |
| 7 | $n-C_{12}H_{25}$ | $-CH_2-C_6H_5$ | Br | 61 - 65 |
| 8 | $n-C_{12}H_{25}$ | $-CH_2-C_6H_5$ | Cl | |
| 9 | $n-C_{12}H_{25}$ | $-CH_2-(2-Cl-C_6H_4)$ | Cl | 89 |
| 10 | $n-C_{12}H_{25}$ | $-CH_2-(3-Cl-C_6H_4)$ | Cl | |
| 11 | $n-C_{12}H_{25}$ | $-CH_2-(4-Cl-C_6H_4)$ | Cl | 93 |
| 12 | $n-C_{12}H_{25}$ | $-CH_2CH_2O-(2-Cl-C_6H_4)$ | Br | 94 |
| 13 | $n-C_{12}H_{25}$ | $-CH_2CH_2O-(3-Cl-C_6H_4)$ | Br | 146 |
| 14 | $n-C_{12}H_{25}$ | $-CH_2CH_2O-(4-Cl-C_6H_4)$ | Br | 135 |
| 15 | $n-C_{12}H_{25}$ | $-CH_2-CH=CHCl$ | Cl | |

| Nr. | $R^1$ | $R^2$ | X | Fp. [°C] |
|---|---|---|---|---|
| 16 | $n-C_{12}H_{25}$ | $-CH_2-C=CH$ | Br | |
| 17 | $n-C_{12}H_{25}$ | $-CH_2CH=CH-CH_3$ | Cl | |
| 18 | $n-C_{12}H_{25}$ | $-CH_2CCl=CH_2$ | Br | |
| 19 | $n-C_{12}H_{25}$ | $-CH_2CCl=CCl_2$ | Br | |
| 20 | $n-C_{14}H_{29}$ | $CH_3$ | Br | |
| 21 | $n-C_{14}H_{29}$ | $CH_3$ | Cl | |
| 22 | $n-C_{14}H_{29}$ | $CH_3$ | I | 91 - 95 |
| 23 | $n-C_{14}H_{29}$ | $-CH_2C_6H_5$ | Br | 129 |
| 24 | $n-C_{14}H_{29}$ | $-CH_2C_6H_5$ | Cl | |
| 25 | $n-C_{14}H_{29}$ | $-CH_2-(2-Cl-C_6H_4)$ | Cl | 92 |
| 26 | $n-C_{14}H_{29}$ | $-CH_2-(3-Cl-C_6H_4)$ | Cl | |
| 27 | $n-C_{14}H_{29}$ | $-CH_2-(4-Cl-C_6H_4)$ | Cl | 115 |
| 28 | $n-C_{14}H_{29}$ | $-CH_2CH_2O-(2-Cl-C_6H_4)$ | Br | 97 |
| 29 | $n-C_{14}H_{29}$ | $-CH_2CH_2O-(3-Cl-C_6H_4)$ | Br | 144 |
| 30 | $n-C_{14}H_{29}$ | $-CH_2CH_2O-(4-Cl-C_6H_4)$ | Br | 133 |
| 31 | $n-C_{14}H_{29}$ | $-CH_2-CH=CH_2$ | Cl | |
| 32 | $n-C_{14}H_{29}$ | $-CH_2-CH=CH_2$ | Br | 83 |
| 33 | $n-C_{14}H_{29}$ | $-CH_2-CH=CHCl$ | Cl | |
| 34 | $n-C_{14}H_{29}$ | $-CH_2-CH=CH-CH_3$ | Cl | |

| Nr. | $R^1$ | $R^2$ | X | Fp. [°C] |
|---|---|---|---|---|
| 35 | $n-C_{14}H_{29}$ | $-CH_2-CCl=CCl_2$ | Cl | |
| 36 | $n-C_{14}H_{29}$ | Propargyl | Br | |
| 37 | $n-C_{14}H_{29}$ | Propargyl | Cl | |
| 38 | $n-C_{16}H_{33}$ | $CH_3$ | Br | |
| 39 | $n-C_{16}H_{33}$ | $CH_3$ | Cl | |
| 40 | $n-C_{16}H_{33}$ | $CH_3$ | I | 96 - 101 |
| 41 | $n-C_{16}H_{33}$ | $-CH_2C_6H_5$ | Br | 57 - 60 |
| 42 | $n-C_{16}H_{33}$ | $-CH_2C_6H_5$ | Cl | |
| 43 | $-(CH_2)_5-O-CH_2CH(C_2H_5)CH_2CH(CH_3)_2$ | $CH_3$ | Br | |
| 44 | $-(CH_2)_5-O-CH_2CH(C_2H_5)CH_2CH(CH_3)_2$ | $CH_3$ | I | |
| 45 | $-(CH_2)_5-O-CH_2CH(C_2H_5)CH_2CH(CH_3)_2$ | $-CH_2CH=CH_2$ | Br | |
| 46 | $-(CH_2)_5-O-CH_2CH(C_2H_5)CH_2CH(CH_3)_2$ | $-CH_2C_6H_5$ | Br | Öl |
| 47 | $-(CH_2)_5-O-CH_2CH(C_2H_5)CH_2CH(CH_3)_2$ | $-CH_2CH_2O-C_6H_5$ | Br | |
| 48 | $-(CH_2)_6-O-CH_2CH(CH_3)CH_2CH(CH_3)_2$ | $CH_3$ | I | |
| 49 | $-(CH_2)_6-O-CH_2CH(CH_3)CH_2CH(CH_3)_2$ | $CH_3$ | Br | |
| 50 | $-(CH_2)_6-O-CH_2CH(CH_3)CH_2CH(CH_3)_2$ | $-CH_2-C_6H_5$ | Br | |
| 51 | $-(CH_2)_6-O-CH_2CH(CH_3)CH_2CH(CH_3)_2$ | $-CH_2-C_6H_5$ | Cl | |
| 52 | $-(CH_2)_6-O-CH_2CH(CH_3)CH_2CH(CH_3)_2$ | $-CH_2CH_2O-C_6H_5$ | Br | |
| 53 | $-(CH_2)_6-O-CH_2CH(CH_3)CH_2CH(CH_3)_2$ | $-CH_2CH=CH_2$ | Br | |
| 54 | $-(CH_2)_6-O-CH(CH_3)CH_2CH_2CH(C_2H_5)_2$ | $-CH_3$ | Br | |
| 55 | $-(CH_2)_6-O-CH(CH_3)CH_2CH_2CH(C_2H_5)_2$ | $-CH_3$ | I | |

| Nr. | $R^1$ | $R^2$ | X | Fp. [°C] |
|---|---|---|---|---|
| 56 | $-(CH_2)_6-O-CH(CH_3)CH_2CH_2CH(C_2H_5)_2$ | $-CH_2C_6H_5$ | Br | |
| 57 | $-(CH_2)_6-O-CH(CH_3)CH_2CH_2CH(C_2H_5)_2$ | $-CH_2C_6H_5$ | Cl | |
| 58 | $-(CH_2)_6-O-CH(CH_3)CH_2CH_2CH(C_2H_5)_2$ | $-CH_2CH_2OC_6H_5$ | Br | |
| 59 | $-(CH_2)_6-O-CH(CH_3)CH_2CH_2CH(C_2H_5)_2$ | $-CH_2CH=CH_2$ | Br | |
| 60 | $-(CH_2)_5-O-CH_2CH_2CH(C_2H_5)(CH_2)_3CH_3$ | $-CH_3$ | I | |
| 61 | $-(CH_2)_5-O-CH_2CH_2CH(C_2H_5((CH_2)_3CH_3$ | $-CH_3$ | Br | |
| 62 | $-(CH_2)_5-O-CH_2CH_2CH(C_2H_5)(CH_2)_3CH_3$ | $-CH_2C_6H_5$ | Cl | |
| 63 | $-(CH_2)_5-O-CH_2CH_2CH(C_2H_5)(CH_2)_3CH_3$ | $-CH_2C_6H_5$ | Br | |
| 64 | $-(CH_2)_5-O-CH_2CH_2CH(C_2H_5)(CH_2)_3CH_3$ | $-CH_2CH=CH_2$ | Br | |
| 65 | $-(CH_2)_5-O-CH_2CH_2CH(C_2H_5)(CH_2)_3CH_3$ | $-CH_2CH_2O-C_6H_5$ | Br | |
| 66 | $-(CH_2)_6-O-(CH_2)_2CH(CH_3)(CH_2)_3CH(CH_3)_2$ | $CH_3$ | Br | |
| 67 | $-(CH_2)_6-O-(CH_2)_2CH(CH_3)(CH_2)_3CH(CH_3)_2$ | $CH_3$ | I | |
| 68 | $-(CH_2)_6-O-(CH_2)_2CH(CH_3)(CH_2)_3CH(CH_3)_2$ | $-CH_2C_6H_5$ | Cl | |
| 69 | $-(CH_2)_6-O-(CH_2)_2CH(CH_3)(CH_2)_3CH(CH_3)_2$ | $-CH_2C_6H_5$ | Br | Öl |
| 70 | $-(CH_2)_6-O-(CH_2)_2CH(CH_3)(CH_2)_3CH(CH_3)_2$ | $-CH_2CH=CH_2$ | Br | |
| 71 | $-(CH_2)_6-O-(CH_2)_2CH(CH_3)(CH_2)_3CH(CH_3)_2$ | $-CH_2CH_2O-C_6H_5$ | Br | |
| 72 | $-(CH_2)_4-O-(CH_2)_2CH(CH_3)(CH_2)_3CH(CH_3)(CH_2)_3CH(CH_3)_2$ | $CH_3$ | Br | |
| 73 | $-(CH_2)_4-O-(CH_2)_2CH(CH_3)(CH_2)_3CH(CH_3)(CH_2)_3CH(CH_3)_2$ | $CH_3$ | I | |
| 74 | $-(CH_2)_4-O-(CH_2)_2CH)CH_3)(CH_2)_3CH(CH_3)(CH_2)_3CH(CH_3)_2$ | $-CH_2C_6H_5$ | Br | |
| 75 | $-(CH_2)_4-O-(CH_2)_2CH)CH_3)(CH_2)_3CH(CH_3)(CH_2)_3CH(CH_3)_2$ | $-CH_2C_6H_5$ | Cl | |
| 76 | $-(CH_2)_4-O-(CH_2)_2CH)CH_3)(CH_2)_3CH(CH_3)(CH_2)_3CH(CH_3)_2$ | $-CH_2CH=CH_2$ | Br | |
| 77 | $-(CH_2)_4-O-(CH_2)_2CH)CH_3)(CH_2)_3CH(CH_3)(CH_2)_3CH(CH_3)_2$ | $-CH_2CH_2O-C_6H_5$ | Br | |
| 78 | $-(CH_2)_4O-(2-CH(CH_3)_2-5-CH_3-cycl.C_6H_9)$ | $CH_2C_6H_5$ | Br | 135 |
| 79 | $-(CH_2)_4O-(2-CH(CH_3)_2-5-CH_3-cycl.C_6H_9)$ | $CH_3$ | Br | |

| Nr. | $R^1$ | $R^2$ | X | Fp. [°C] |
|---|---|---|---|---|
| 80 | $-(CH_2)_4O-(2-CH(CH_3)_2-5-CH_3-cycl.C_6H_9)$ | $CH_3$ | I | |
| 81 | $-(CH_2)_4O-(2-CH(CH_3)_2-5-CH_3-cycl.C_6H_9)$ | $-CH_2-CH=CH_2$ | Br | |
| 82 | $-(CH_2)_6-O-(4-tert.-C_4H_9-cycl.C_6H_{10})$ | $-CH_3$ | Br | |
| 83 | $-(CH_2)_6-O-(4-tert.C_4H_9-cycl.C_6H_{10})$ | $-CH_3$ | I | 96 |
| 84 | $-(CH_2)_6-O-(4-tert.C_4H_9-cycl.C_6H_{10})$ | $-CH_2C_6H_5$ | Br | 116 |
| 85 | $-(CH_2)_6-O-(4-tert.C_4H_9-cycl.C_6H_{10})$ | $-CH_2C_6H_5$ | Cl | |
| 86 | $-(CH_2)_6-O-(4-tert.C_4H_9-cycl.C_6H_{10})$ | $-CH_2CH=CH_2$ | Br | |
| 87 | $-(CH_2)_4-O-CH_2CH(CH_3)CH_2-C_6H_5$ | $-CH_3$ | I | 81 |
| 88 | $-(CH_2)_4-O-CH_2CH(CH_3)CH_2-C_6H_5$ | $-CH_3$ | Br | |
| 89 | $-(CH_2)_4-O-CH_2CH(CH_3)CH_2-C_6H_5$ | $-CH_2C_6H_5$ | Br | 110 |
| 90 | $-(CH_2)_4-O-CH_2CH(CH_3)CH_2-C_6H_5$ | $-CH_2C_6H_5$ | Cl | |
| 91 | $-(CH_2)_4-O-CH_2CH(CH_3)CH_2-C_6H_5$ | $-CH_2CH=CH_2$ | Br | |

Charakteristische $^{1}H$-NMR-Daten

| Verbindung Nr. | Lösungsmittel | Chem. Verschiebung in ppm |
|---|---|---|
| 96 | $d_6$-DMSO | s 5,9 [24]; verbr. dd 8,15 [1H]; verbr. d 8,30 [14], d 8,90 [1H]; s 9,25 [1H] |
| 119 | $d_6$-DMSO | s 5,92 [2H]; dd 8,10 [1H]; verbr. d 8,28 [1H]; d 8,92 [1H]; s 9,28 [1H] |
| 137 | $d_6$-DMSO | s 4,35 [3H]; dd 8,05 [1H]; verbr. d 8,20 [1H]; d 8,62 [1H]; s 8,92 [1H] |

Die neuen Verbindungen eignen sich zur Bekämpfung von Algen, Bakterien oder Pilzen außer im Pflanzenschutz beispielsweise zur Desinfektion des Wassers in Luftbefeuchtungsanlagen, Kühlwasserkreisläufen, Wasserinjektionssystemen und für Erdölfelder, Schwimmbäder, Wäschereien sowie in Präparaten, die zur Desinfektion im Lebensmittelbereich, Molkereien, Brauereien, Krankenhäusern eingesetzt werden. Weiterhin ist die Behandlung von Rohhäuten und Leder zu nennen.

Die mikrobiziden Mittel enthalten im allgemeinen zwischen O,1 und 95, vorzugsweise zwischen O,5 und 9O Gew.-% Wirkstoff.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung oder wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Ethanolamin, Dimethylformamid) und Wasser; Trägerstoffe wie natürliche Gesteinsmehle, z.B. Kaoline, Tonerden, Talkum, Kreide und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die Wirkstoffe wirken für sich allein als schaumarme Biozide. Eine bedeutende Steigerung der Wirkung dieser Verbindungen enthaltender biozider Zubereitungen wird erzielt, wenn man ihnen noch Tri-$C_6$- bis $C_{12}$-alkylmethylammoniumsalze, vorzugsweise in Mengen von 2O - 4O Gew.-%, bezogen auf das Gewicht der Verbindungen der allgemeinen Formel I, zusetzt.

Die erfindungsgemäß in den Zubereitungen enthaltenden Verbindungen sind gegen eine große Zahl von Mikroorganismen wirksam, und es seien beispielhaft angegeben:
Staphylococcus aureus, Escherichia coli, Klebsiella pneumoniae, Citrobacter freundii, Proteus vulgaris, Pseudomonas aeruginosa, Desulfovibrio desulfuricans, Streptoverticillium rubrireticuli, Aspergillus niger, Aspergillus versicolor, Penicillium funiculosum, Penicillium expansum, Penicillium glaucum, Paecilomyces variotii, Trichoderma viride, Chaetonium globosum, Candida albicans, Trichophyton mentagrophytes, Geotrichum candidans, Monilia sitophila, Scenedesmus quadricauda, Chlorella vulgaris, Nostoc muscorium, Oscillatoria limosa und Anabaena constricta.

Als übliche Anwendungskonzentration wählt man - bezogen auf das Gewicht des zu schützenden Materials - O,O1 - 2 % an Wirkstoff; beim Einsatz zur Wasserbehandlung, bei der Erdölförderung, in Schwimmbädern, Rückkühlwerken, Luftbefeuchtungsanlagen oder in der Papierindustrie sind Wirkstoffmengen von 5 - 25O ppm ausreichend. Gebrauchsfertige Desinfektionsmittellösungen enthalten z.B. O,5 - 1O Gew.-% an Wirkstoff. Die Wirkstoffe können auch mit anderen bekannten Mikroziden gemischt werden. In vielen Fällen erhält man dabei einen synergistischen Effekt.

Solche Wirkstoffe sind beispielsweise:

2-(Thiocyanomethylthio)-benzthiazol
1-[2-(2,4-Dichlorphenyl)-2-(2-propenyl-oxy)-ethyl]-1H-imidazol
2,4,5,6-Tetrachlor-isophthalodinitril
Methylenbisthiocyanat
Tributylzinnoxid, -naphthenat, -benzoat, -salicylat
Mercaptobenzthiazol
1,2-Benzisothiazolon und seine Alkalisalze
Alkaliverbindungen des N'-Hydroxy-N-cyclohexyl-diazeniumoxids
2-(Methoxy-carbonylamino)-benzimidazol
2-Methyl-3-oxo-5-chlor-thiazolin-3-on
Trihydroxymethyl-nitro-methan
Glutardialdehyd
Chloracetamid
Polyhexamethylenbisguanide
5-Chlor-2-methyl-4-isothiazolin-3-on + Magnesiumsalze
3,5-Dimethyltetrahydro-1,3,5-2H-thiadiazin-2-thion
Hexahydrotriazin
N,N-Methylolchloracetamid
2-n-Octyl-4-isothiazol-in-3-on
Oxazolidine
Bisoxazolidine
2,5-Dihydro-2,5-dialkoxy-2,5-dialkylfurane
Diethyl-dodecyl-benzyl-ammoniumchlorid
Dimethyl-octadecyl-dimethylbenzyl-ammoniumchlorid
Dimethyl-didecyl-ammoniumchlorid
Dimethyl-didodecyl-ammoniumchlorid
Trimethyl-tetradecylammoniumchlorid
Benzyl-dimethyl-alkyl-($_{12}$-$C_{18}$)-ammoniumchlorid
Dichlorbenzyl-dimethyl-dodecyl-ammoniumchlorid
Cetylpyridiniumchlorid
Cetylpyridiniumbromid
Cetyl-trimethyl-ammoniumchlorid
Laurylpyridiniumchlorid
Laurylpyridiniumbisulfat
Benzyl-dodecyl-di(beta-oxyethyl)-ammoniumchlorid
Dodecylbenzyl-trimethyl-ammoniumchlorid
n-Alkyl-dimethyl-benzyl-ammoniumchlorid
(Alkylrest: 40 % $C_{12}$, 50 % $C_{14}$, 10 % $C_{16}$)
Lauryl-dimethyl-ethyl-ammoniumethylsulfat
n-Alkyl-dimethyl-(1-naphthylmethyl)-ammoniumchlorid
(Alkylrest: 98 % $C_{12}$, 2 % $C_{14}$)
Cetyldimethylbenzylammoniumchlorid
Lauryldimethylbenzylammoniumchlorid

Die mikrobiziden Mittel enthalten im allgemeinen zwischen 0,1 und 95, vorzugsweise zwischen 0,5 und 90 Gew.-% Wirkstoff.

<u>Anwendungsbeispiel 1</u>

Algizide Wirksamkeit gegenüber Grünalgen

Zur Prüfung der Wirksamkeit gegenüber Grünalgen werden die Wirkstoffe einer für die Vermehrung der einzelligen Grünalge Chlorella vulgaris begünstigenden, phosphatreichen Nährlösung in Mengen von 10; 7,5; 5; 2,5 und 1 Gew.-Teilen pro Million Teile Nährlösung zugesetzt. Jeweils 100 ml Nährlösungswirkstoffgemisch sowie nur Nährlösung (Kontrolle) werden in 300 ml-Erlenmeyerkolben gegeben. Die Nährlösung wird vor dem Wirkstoffzusatz mit einer Suspension der Alge Chlorella vulgaris beimpft; die Zelldichte wird auf $10^6$ Zellen/ml Nährlösung eingestellt. Nach 14-tägiger Aufstellung der Versuchsansätze bei Raumtemperatur und Lichtzutritt wird die Wirksamkeit beurteilt.

Das Ergebnis des Versuches zeigt, daß die Verbindungen 6, 7, 9, 11, 25, 27, 32, 40, 46, 50, 56, 64, 69 und 78 bei der Anwendung von 10; 7,5; 5; 2,5 und 1 Gew.-Teilen Wirkstoff je Million Teile Nährlösung die Algen gut bekämpfen.

Anwendungsbeispiel 2

Fungizide Wirksamkeit gegenüber Aspergillus niger

Die Wirkstoffe werden einer für das Wachstum des Pilzes Aspergillus niger optimal geeigneten Nährlösung in Mengen von 100, 75, 50, 25 und 10 Gew.-Teilen pro Million Teile Nährlösung zugesetzt. Es werden jeweils 20 ml der so behandelten Nährlösung in 100 ml Glaskolben mit 0,3 mg Aspergillus-Pilzsporen beimpft. Die Kolben werden 120 Stunden lang bei 36 °C erwärmt und anschließend das Ausmaß der Pilzentwicklung, das bevorzugt auf der Nährlösungsoberfläche erfolgt, beurteilt.

Das Ergebnis des Versuches zeigt, daß die Verbindungen 6, 7, 9, 11, 13, 14, 22, 23, 25, 27, 28, 30, 32 und 40 bei der Anwendung von 100, 75, 50, 25 und 10 Gew.-Teilen Wirkstoff je Million Teile Nährlösung eine gute fungizide Wirkung haben (90 %).

Anwendungsbeispiel 3

Wirksamkeit gegenüber Trichophyton mentagrophytes und Candida albicans

Zur Prüfung der Wirksamkeit gegenüber Pilzen werden die Wirkstoffe einer für das Wachstum der Pilze Trichophyton mentagrophytes und Candida albicans geeigneten Nährlösung in Mengen von 100, 50, 25, 12, 6, 3 und 1,5 Gew.-Teilen pro Million Teile Nährlösung zugesetzt. Jeweils 10 ml Nährlösungs-Wirkstoffgemisch werden in sterile Reagenzgläser gegeben und mit einem Tropfen einer Sporensuspension beimpft, die $10^6$ Konidien bzw. Zellen enthält. Nach 120-stündiger Bebrütung werden aus denjenigen Röhrchen, die kein sichtbares Pilzwachstum zeigen, Proben entnommen und auf Pilznährböden übertragen. Es wird die Verdünnungsstufe festgestellt, bei welcher nach dem Übertragen einer Probe auf den Nährboden noch kein Wachstum der Pilze erfolgt.

Das Ergebnis des Versuches zeigt, daß die Verbindungen 4, 5, 6, 7, 9, 11, 12, 13, 14, 22, 23, 25, 27, 28, 29, 30, 32, 40, 41, 46, 50, 56, 64, 69 und 87 bei der Anwendung von 1,5 bis 25 ppm eine gute fungizide Wirkung haben.

Anwendungsbeispiel 4

Bakterizide Wirksamkeit gegenüber Staphylococcus aureus, Escherichia coli und Proteus vulgaris

Die Abtötungswerte gegenüber Bakterien wurden wie folgt ermittelt: Zu je 5 ml einer Verdünnung der Mittel in Wasser wurden 5 ml doppeltkonzentrierter Nährbouillon in sterile Reagenzgläser gegeben und vermischt. Die Versuchsansätze enthalten 200, 100, 50, 25, 12, 6 und 3 Gew.-Teile Wirkstoff pro Million Teile Nährbouillon. Durch Zugabe von einem Tropfen 1:10 verdünnter 16 Stunden alter Bouillon-Kulturen der Bakterienarten Staphylococcus aureus, Escherichia coli bzw. Proteus vulgaris wurden dann die Röhrchen beimpft und 24 Stunden lang bei 37°C bebrütet. Nach dieser Zeit wurden Proben aus den Röhrchen auf Bakteriennährböden übertragen und diese ebenfalls 24 Stunden lang bei 37°C bebrütet. Diejenige Verdünnungsstufe, bei welcher nach dem Übertragen einer Probe auf den Nährboden keine Bakterienentwicklung mehr erfolgte, wird festgestellt.

Das Ergebnis des Versuchs zeigt, daß die Verbindungen 4, 5, 6, 7, 11, 14 und 23 bei der Anwendung von 6 bis 50 ppm Wirkstoff eine gute fungizide Wirkung haben.

Anwendungsbeispiel 5

Wirksamkeit gegenüber den Pilzen Paecilomyces variotii, Aureobasidium pullulans, Geotrichum candidans

Zur Prüfung der Wirksamkeit gegenüber Pilzen werden die Wirkstoffe einer für das Wachstum der Pilze Paecilomyces variotii, Aureobasidium pullulans und Geotrichum candidans geeigneten Nährlösung in Mengen von 100, 50, 25, 12, 6, 3 und 1,5 Gew.-Teilen pro Million Teile Nährlösung zugesetzt. Jeweils 10 ml Nährlösungs-Wirkstoffgemisch werden in sterile Reagenzgläser gegeben und mit einem Tropfen einer Sporensuspension beimpft, die $10^6$ Konidien bzw. Zellen enthält. Nach 120-stündiger Bebrütung werden aus denjenigen Röhrchen, die kein sichtbares Pilzwachstum zeigen, Proben entnommen und auf Pilznährböden übertragen. Es wird die Verdünnungsstufe festgestellt, bei welcher nach dem Übertragen einer Probe auf den Nährboden kein Wachstum der Pilze mehr erfolgt.

Das Ergebnis des Versuches zeigt, daß die Verbindungen 4, 5, 6, 7, 9, 11, 12, 13, 14, 22, 23, 25, 26, 28, 29, 30, 32, 40, 41, 46, 56 und 78 bei der Anwendung von 1,5 bis 12 ppm Wirkstoff eine gute fungizide Wirkung zeigen.

**Patentansprüche**

1. Mittel zur Bekämpfung von Algen, Bakterien oder Pilzen außer bei Pflanzen, enthaltend einen festen oder flüssigen Trägerstoff und ein O-substituiertes 3-Oxypyridiniumsalz der Formel

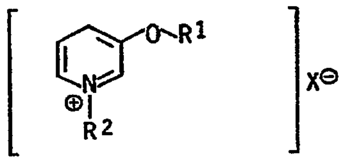
I,

in der
$R^1$ Dodecyl, Tetradecyl, Hexadecyl oder die Gruppe $C_{2-10}$-Alkyl-O-$C_{5-18}$-Alkyl mit insgesamt 10 bis 20 C-Atomen,
$R^2$ Methyl, Allyl oder die Reste $-(CH_2)_{1-2}$-Ar oder $-(CH_2)_{2-6}$OAr bedeutet,
wobei Ar für Phenyl oder einfach bis dreifach durch Halogen substituiertes Phenyl steht und
$X^-$ $Cl^-$, $B^-$ oder $I^-$ oder ein Anion, das ein Äquivalent einer nicht phytotoxischen Säure ist, bedeutet.

2. Verfahren zur Bekämpfung von Algen, Bakterien oder Pilzen außer bei Pflanzen, dadurch gekennzeichnet, daß man die Mikroorganismen oder die durch Befall durch die Mikroorganismen bedrohten Gegenstände mit einer toxischen Menge eines O-substituiertes 3-Oxypyridiniumsalz der Formel

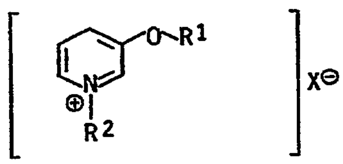
I,

in der
$R^1$ Dodecyl, Tetradecyl, Hexadecyl oder die Gruppe $C_{2-10}$-Alkyl-O-$C_{5-18}$-Alkyl mit insgesamt 10 bis 20 C-Atomen,
$R^2$ Methyl, Allyl oder die Reste $-(CH_2)_{1-2}$-Ar oder $-(CH_2)_{2-6}$OAr bedeutet,
wobei Ar für Phenyl oder einfach bis dreifach durch Halogen substituiertes Phenyl steht und
$X^-$ $Cl^-$, $B^-$ oder $I^-$ oder ein Anion, das ein Äquivalent einer nicht phytotoxischen Säure ist, bedeutet, behandelt.

3. Verfahren gemäß Anspruch 2, dadurch gekennzeichnet, daß man eine Verbindung verwendet, in der $R^1$ Dodecyl, $R^2$ Allyl, Benzyl oder Monochlorbenzyl und X Chlorid oder Bromid bedeutet.

4. Verfahren zur Herstellung eines mikroziden Mittels gemäß Anspruch 1, dadurch gekennzeichnet, daß man ein O-substituiertes 3-Oxypyridiniumsalz definiert wie in Anspruch 1 mit einem festen oder flüssigen Trägerstoff sowie gegebenenfalls mit einem oder mehreren oberflächenaktiven Mitteln mischt.

**Claims**

1. An agent for controlling algae, bacteria or fungi, except in the case of plants, containing a solid or liquid carrier and an O-substituted 3-oxypyridinium salt of the formula

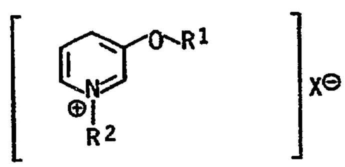
I,

where $R^1$ is dodecyl, tetradecyl, hexadecyl or a group $C_2$–$C_{10}$-alkyl–O–$C_5$–$C_{18}$-alkyl having a total of 10 to 20 carbon atoms, $R^2$ is methyl, allyl or the radical $-(CH_2)_{1-2}$-Ar or $-(CH_2)_{2-6}$OAr, Ar being phenyl which is unsubstituted or monosubstituted to trisubstituted by halogen, and $X^-$ is $Cl^-$, $Br^-$ or $I^-$ or an anion which is one equivalent of a nonphytotoxic acid.

2. A method for controlling algae, bacteria or fungi, except in the case of plants, wherein the microorganisms or the articles threatened by attack by the microorganisms are treated with a toxic amount of an O-substituted 3-oxypyridinium salt of the formula

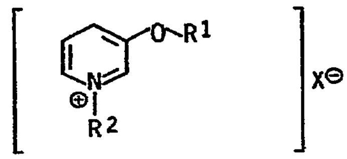

I,

where $R^1$ is dodecyl, tetradecyl, hexadecyl or a group $C_2$–$C_{10}$-alkyl–O–$C_5$–$C_{18}$-alkyl having a total of 10 to 20 carbon atoms, $R^2$ is methyl, allyl or the radical $-(CH_2)_{1-2}$-Ar or $-(CH_2)_{2-6}$OAr, Ar being phenyl which is unsubstituted or monosubstituted to trisubstituted by is halogen, and $X^-$ is $Cl^-$, $Br^-$ or $I^-$ or an anion which is one equivalent of a nonphytotoxic acid.

3. A method as claimed in claim 2, wherein a compound in which $R^1$ is dodecyl and $R^2$ is allyl, benzyl or monochlorobenzyl and X is chloride or bromide is used.

4. A process for the preparation of a microcidal agent as claimed in claim 1, wherein an O-substituted 3-oxypyridinium salt defined as in claim 1 is mixed with a solid or liquid carrier and, if required, with one or more surfactants.

**Revendications**

1. Agent de lutte contre les algues, bactéries ou champignons, en dehors des plantes, contenant un support solide ou liquide et un sel de 3-oxypyridinium substitué sur O, de la formule

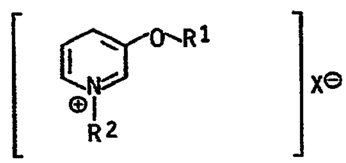

I,

dans laquelle
$R^1$ représente dodécyle, tétradécyle, héxadécyle ou le groupe (alkyle en $C_{2-10}$)–O–alkyle en $C_{5-18}$ ayant au total 10 à 20 atomes C
$R^2$ représente méthyle, alkyle ou le reste $-(CH_2)_{1-2}$-Ar ou $-(CH_2)_{2-6}$–O–Ar
Ar étant mis pour phényle ou phényle substitué une à trois fois par halogène et
$X^-$ représente $Cl^-$, $Br^-$ ou $I^-$ ou un anion qui est un équivalent d'un acide non phytotoxique.

2. Procédé de lutte contre les algues, bactéries ou champignons, en dehors des plantes, caractérisé par le fait que l'on traite les microorganismes, ou les objets menacés d'une attaque par les microorganismes, avec une quantité toxique d'un sel de 3-oxypyridinium substitué sur O, de la formule

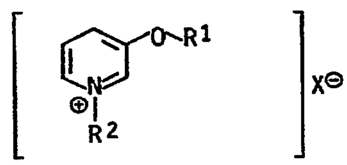

I,

dans laquelle
$R^1$ représente dodécyle, tétradécyle, héxadécyle ou le groupe (alkyle en $C_{2-10}$)–O–alkyle en $C_{5-18}$ ayant au total 10 à 20 atomes C
$R^2$ représente méthyle, alkyle ou le reste $-(CH_2)_{1-2}$-Ar ou $-(CH_2)_{2-6}$–O–Ar
Ar étant mis pour phényle ou phényle substitué une à trois fois par halogène et
$X^-$ représente $Cl^-$, $Br^-$ ou $I^-$ ou un anion qui est un équivalent d'un acide non phytotoxique.

3. Procédé selon la revendication 2, caractérisé par le fait qu'on utilise un composé dans lequel $R^1$ représente dodécyle, $R^2$, alkyle, benzyle ou monochlorobenzyle et X chlorure ou bromure.

4. Procédé de préparation d'un agent microbicide selon la revendication 1, caractérisé par le fait que l'on mélange un sel de 3-oxypyridine substitué en O, défini comme dans la revendication 1, avec un support solide ou liquide ainsi qu'avec éventuellement un ou plusieurs agents tensio-actifs.